# EUROPEAN PATENT APPLICATION

(11) **EP 1 355 252 A1**
(43) Date of publication of application: **22.10.2003**
(21) Application number: 03252135.3
(22) Date of filing: 03.04.2003
(51) Int. Cl.: G06F 17/60

(54) **Prescription pharmaceutical labeling system**

(30) Priority: 15.04.2002 US 123578
(71) Applicant: Hewlett-Packard Company, Palo Alto, CA 94304 (US)
(72) Inventor: Valley, Jeffrey M., Corvallis, OR 97330 (US); Greeven, John C., Corvallis, OR 97330 (US)
(74) Representative: Jackson, Richard Eric

(57) **Abstract**

A method of providing a pharmaceutical label having an identifying image (32) printed thereon is provided. The method includes receiving (104) a request to fill a patient prescription for a pharmaceutical, accessing an identification database (106, 110) including identifying images, retrieving a prescription-specific identifying image (108, 112) from the identification database, and printing a label (114) including the prescription-specific identifying image.

## Description

### BACKGROUND OF THE INVENTION

A significant number of prescriptions and prescription refills are inaccurately filled annually. Inaccurately filled prescriptions may be caused by difficult-to-read handwriting on prescriptions, confusion between drugs with similar names, and confusion between drugs with similar appearances. For example, an error may result from a mistake in reading the prescribing physician's handwriting. The prescription container thus may be filled with a drug that has a name that sounds or looks like the prescribed drug, but is really the wrong drug. Other errors in filling prescriptions may be the result of a confusion between two patients having similar names, either at the time of filing the prescription or at the time of use. An inaccurately or incorrectly filled or used prescription has the potential to cause severe injury or even death in some cases.

### SUMMARY OF THE INVENTION

A method of providing a pharmaceutical label having an identifying image printed thereon is provided. The method includes receiving a request to fill a patient prescription for a pharmaceutical, accessing an identification database including identifying images, retrieving a prescription-specific identifying image from the identification database, and printing a label including the prescription-specific identifying image.

### BRIEF DESCRIPTION OF THE DRAWINGS

Fig. 1 is a somewhat schematic representation of a system configured to create prescription pharmaceutical labels with identifying images thereon according to an embodiment of the present invention.
Fig. 2 is a prescription pharmaceutical label according to an embodiment of the present invention.
Fig. 3 is a perspective view of a prescription pharmaceutical container including the label of Fig. 2.
Fig. 4 is a flow chart illustrating a method of verifying that a prescription has been filled accurately, according to an embodiment of the present invention.

### DETAILED DESCRIPTION OF THE PREFERRED EMBODIMENT

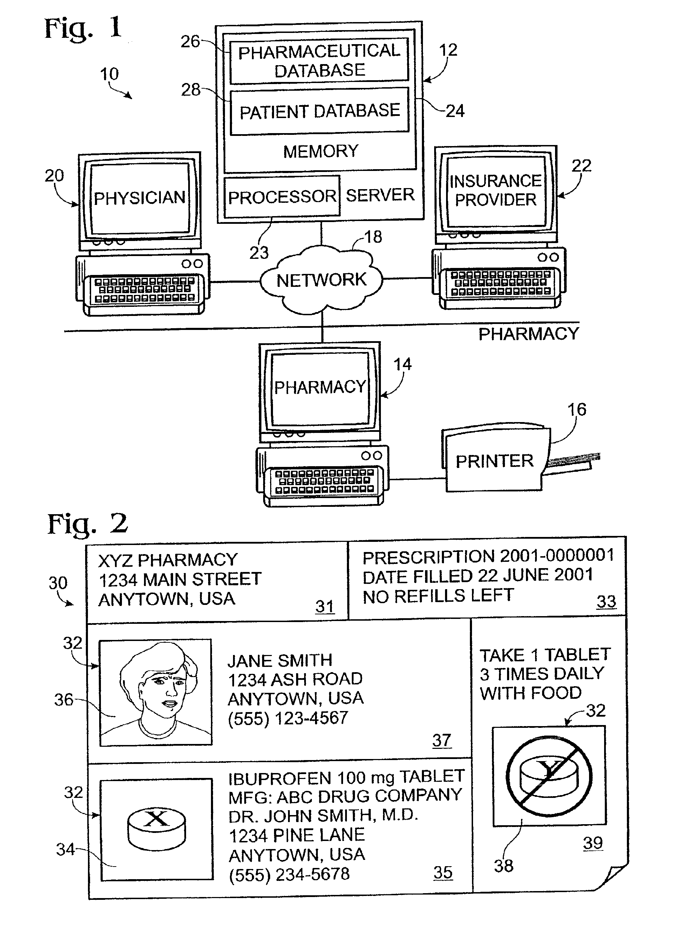

Referring initially to Fig. 1, a system configured to implement the method of the present invention is indicated generally at 10. System 10 may include a server 12, a pharmacy terminal 14, a printer 16, and a network communications link 18. Additionally, system 10 may include a physician terminal 20 and/or an insurance provider terminal 22, either or both of which may be used for entering patient information and communicating the information to server 12, or any of the other terminals.

Server 12 includes a processor 23 and memory 24. Processor 23 may be configured to accommodate identification of a prescription pharmaceutical transaction, and to access memory 24 to retrieve a prescription-specific identifying image as will be described further below. The server may be any suitable network server computer configured to store data and to be accessed from a proximate or remote terminal or terminals. A single computer or a set of networked computers may function as the server. Access to server 12 may be through a graphical user interface on the server itself or may be through one or more of the designated terminals.

As indicated, a pharmaceutical database 26 may be stored in memory 24 of server 12. A patient database 28 may also be stored in memory 24 of server 12. Either pharmaceutical database 26 or patient database 28 similarly may be stored in memory of one or more of the designated terminals. Additionally, either pharmaceutical database 26, or patient database 28, may be stored on separate servers at proximate or disparate locations. Furthermore, there may be more than one patient database 26 stored on server 12, or any of the designated terminals. For example, each of a plurality of physicians may maintain a physician-specific patient database.

Pharmacy terminal 14 typically is located at a pharmacy in order to aid the pharmacist in record-keeping, communicate with server 12 and/or the other terminals, and produce custom prescription labels. A typical pharmacy terminal may be a computer terminal including a processor, memory, a user interface, etc. The pharmacy terminal typically permits a pharmacist to access server 12 and to retrieve information stored on the server for use in production of a custom pharmaceutical label. Additionally, other terminals may be accessed from pharmacy terminal 14. Data may be communicated between pharmacy terminal 14 and server 12, or any of the other terminals via network communications link 18, or some other appropriate communications link. Typically, a pharmacist records pharmaceutical transactions using pharmacy terminal 14. A pharmaceutical transaction, as used herein, includes the filling of a specific prescription for a specific patient.

A printer 16 maybe connected to pharmacy terminal 14 for printing custom prescription labels. Typically, printer 16 is configured to handle self-adhesive label blanks, but various forms of media may be employed. Printer 16 may be a laser printer, an ink-jet printer, a dot matrix printer, etc. and may be capable of printing in color or printing only monochromatic images. Printer 16 may be directly connected to pharmacy terminal 14, but also may be linked through a local area network or similar remote communications link. Printer 16 also may be configured to handle multiple sizes of media and to print labels directly on a container or packaging, and may be configured to print a custom label for each pharmaceutical transaction a pharmacy makes.

Physician terminal 20 and/or insurance provider's terminal 22 may communicate data to server 12 for storage. Data communicated may include patient-specific information such as name, address, age, sex, height, weight, known allergies, and a patient identifying image, etc. Typically, a physician terminal may be used to store detailed patient information, including that listed above among other information relevant to a physician's medical practice. The physician may keep records of every patient treated. The physician's records may include patient-specific prescription information and patient identification information, which may be stored on physician terminal 20, or, as noted above, communicated to server 12 or any of the designated terminals. In some instances selective portions of the information from the physician's records may be communicated to server 12. The information communicated may include an identifying image of the patient.

Identifying images of patients may be entered into patient database 28 at the physician's office through the use of a digital camera. Another method of entering identifying images of patients may involve scanning a preexisting photograph of the patient, supplied by the patient. Other procedures for creating a database of identifying images also are available. The physician or one of the physician's assistants or employees may generate an identifying image for each patient the physician treats.

A patient who belongs to an insurance plan that requires use of a health care provider network such as a health maintenance organization (HMO) may have records regarding the patient's medical history stored on an insurance provider's terminal, or a server accessible by HMO care providers. The patient's records may include identification information. For example, the identification information may include an identifying image of the patient. Selective portions of the patient's records, including identification information and the identifying image, may be transmitted to server 12 or other designated terminals. The patient's health insurance provider may enter identifying images of the patient into patient database 28. For example, a recent identification photo may be required of a patient during the insurance application process. The insurance provider may then scan or digitally reproduce the identification photo and include that information in the patient's records. Alternatively, the insurance provider may take a digital photo of the patient.

It will be understood that while terminals 14, 20, and 22 are illustrated and described as computer terminals each may also include a plurality of networked computers. For example, the physician terminal may include multiple computer terminals that may be connected to a local computer network. Similarly, insurance provider terminal 22 may be a network of terminals maintained by the insurance company and accessible by healthcare providers that participate in the insurance company's plan.

Network communications link 18 typically connects server 12, pharmacy terminal 14, physician terminal 20, and insurance provider terminal 22. Network communications link 18 may be any type of network capable of communicating data. For example, network communications link 18 may be a local area network (LAN), a wide area network (WAN), or any combination of these. Network communications link 18 may use any suitable network architecture enabling terminals 14, 20, and 22 to exchange data securely with server 12.

In one embodiment of system 10, pharmacy terminal 14 may include an identifying network address for communicating with server 12. In another embodiment pharmacy terminal 14 may not be able to access server 12 without authorization. Authorization may require a pre-approved user name and/or password, or may require submission of the pharmacist's licensing data. Similarly, physician terminal 20 and insurance provider terminal 22 may require an identifying network address or similar authorization to access server 12 or either of the databases 26 and 28.

One embodiment of the present invention, as shown in Fig. 1, is implemented using a wide area network (WAN) as the network communication link. Server 12 may be linked through WAN 18 to pharmacy terminal 14. Physician terminal 20 and insurance provider terminal 22 also may be linked to server 12 through WAN 18. Each of terminals 14, 20, and 22 may securely transmit and receive data to and from server 12 through WAN 18.

Pharmaceutical database 26 may, for example, include information related to all Food and Drug Administration (FDA) approved pharmaceuticals and/or medications. Among the information included about each FDA approved pharmaceutical and/or medication may be important prescription information such as drug-to-drug interactions, common side effects, black-box warnings, etc. Identifying images of the approved pharmaceuticals and/or medications also may be included in pharmaceutical database 26. The pharmaceutical information, including the identifying images, may be stored in memory 24 of server 12. In particular, such pharmaceutical information may be stored in pharmaceutical database 26. Identifying images included in pharmaceutical database 26 may be photographs or illustrations of pills, capsules, or similar specialized packaging which identifies a prescription pharmaceutical product by its appearance or the appearance of its packaging. Identifying images of pharmaceuticals thus may be referred to as pharmaceutical-identifying images.

Information in pharmaceutical database 26 may need to be kept current as new prescription pharmaceuticals become available. A proprietor of system 10 may handle periodic updating of the database with information and identifying images. For example, when the FDA approves a new prescription pharmaceutical, the relevant prescription information and identifying images for that pharmaceutical may be entered into prescription database 26. Typically, weekly or monthly updates to the prescription database will ensure the information is current. However, the periodic updating may take place more often or less often. It will be understood that any periodic interval for updating the pharmaceutical database may be used in accordance with system 10.

In one embodiment of the invention, the pharmaceutical database may be stored in a specialized server and the patient database may be stored in memory on physician terminal 20. In this embodiment, the prescription the patient gives to the pharmacist may include a code that enables the pharmacist to remotely access the patient database stored on physician terminal 20. This embodiment enables a physician to offer his or her patients a valuable service by maintaining a patient database having limited access.

A label 30 according to one embodiment of the present invention is shown in Fig. 2. Label 30 may include one or more images 32 printed thereon. The images may include a picture or illustration of the pharmaceutical product (e.g. pill or capsule or pharmaceutical packaging), as indicated at 34. Additionally, the images may include a prescription-specific identifying image such as a picture or illustration of the patient who is the recipient of the prescription, as indicated at 36. In either event, a prescription-specific identifying image may convey information relevant to a specific prescription.

Label 30 may include a pharmacy-identification region 31, a prescription-filled region 33, a pharmaceutical-information region 35, a patient-information region 37, and an instructions/warnings region 39. Selected regions on label 30 may include two types of visually cognizable data, prescription-specific identifying images and text. Labels made with this system may include more than one identifying image. Specifically, labels made with this system may include a pharmaceutical-identifying image 34 in pharmaceutical-information region 35 and a patient-identifying image 36 in patient-information region 37.

Pharmacy-identification region 31 of label 30 may include information about the pharmacy filling the prescription, such as the name and address of the pharmacy, as shown in Fig. 2. Additionally, region 31 may include the name of the pharmacist actually filling the prescription and a phone number for the pharmacy or pharmacist.

Prescription-filled region 33 of label 30 may include information about the prescription such as the date the prescription was filled, if any refills are left, and an identifying prescription number.

Pharmaceutical-information region 35 may include a pharmaceutical-identifying image 34, and a textual description of the prescription pharmaceutical. By including a pharmaceutical-identifying image 34, on label 30, a patient and/or pharmacist may verify that the correct prescription pharmaceutical is contained within the prescription container by comparing the contents of the container to pharmaceutical-identifying image 34. It will be understood that third parties other than the patient and pharmacist also may verify the correct prescription was used to fill the prescription container. The textual description of region 35 may include the name of the pharmaceutical, the manufacturer of the pharmaceutical, the prescribing physician's name, the prescribing physician's address, and the prescribing physician's phone number, etc.

Patient-information region 37 may include a prescription-specific identifying image. Such prescription-specific identifying image may be a patient-identifying image 36. Patient-identifying image 36 may be a photographic image of the patient, similar that found on a passport or drivers license. Pharmacists may use patient-identifying image 36 to verify that the correct patient is taking the correct prescription by comparing the patient-identifying image to the person picking up the prescription. Similarly, a patient or other person may verify that the correct patient is taking the correct prescription. For example, when a prescription container is retrieved from a crowded medicine cabinet in a home having many people taking prescription pharmaceuticals, the patient-identifying image printed on label 30 prevents the wrong person from accidentally taking the wrong medication. Additionally, patient-information region 37 may include textual data containing information about a patient, such as the patient's name, address, phone number, etc.

Instruction/warning region 39 may include dosage instructions, warnings about side effects, and other important information about how to take the prescription pharmaceutical. Instruction/warning region 39 also may include a prescription-specific identifying image such as an image of a medication that this patient should not take with the prescribed pharmaceutical. Such a warning-identifying image is shown at 38 in Fig. 2.

With the foregoing in mind, it will be appreciated that a prescription label may be custom made for each prescription pharmaceutical transaction. A prescription pharmaceutical transaction typically occurs when a pharmacist's fills or refills a patient's prescription and dispenses the prescription to the patient. Resulting prescription label 30 may include prescription-specific identifying images printed thereon, as noted above. Such prescription-specific identifying images may be digital images sized to fit on label 30. Prescription-specific identifying images that may be printed on label 30 include, for example, patient-identifying image 36 and warning-identifying image 38. As shown, label 30 includes patient-identifying image 36 placed in patient information region 37. Also as shown, label 30 includes warning-identifying image 38 placed in instruction/warning information region 39.

Therefore, in the embodiment of Fig. 2, a person may verify the accuracy of the prescription contents using pharmaceutical-identifying image 34, the identity of the person taking the prescription using patient-identifying image 36, and any interactions or allergies using warning-identifying image 38. To verify the accuracy of the prescription contents, a person compares the appearance of the contents of the prescription container to the pharmaceutical-identifying image 34, making sure the appearance of the contents matches image 34. To verify the identity of the person receiving the prescription pharmaceutical, a person compares the appearance of the specific person receiving the prescription pharmaceutical to the patient-identifying image 36, making sure the appearance of the person receiving the prescription pharmaceutical matches image 36. Drug interactions, allergies, or the like may be varied by comparing the appearance of any other medications, foods, etc. with any warning-identifying images to make sure there is not a match.

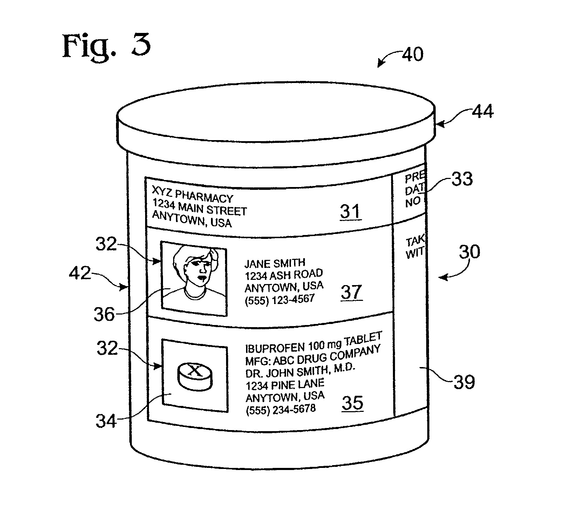

Fig. 3 shows a prescription-pharmaceutical container 40 having prescription-specific label 30 applied thereon. Pharmaceutical container 40 may include a body portion 42 configured to store prescription pharmaceuticals, and a tamper-resistant lid 44 configured to retain the pharmaceuticals within container 40. As shown, label 30 may be applied to the exterior of container 40.

As discussed above, label 30 may include prescription-specific identifying images. When a prescription is filled, a pharmacist may use the identifying images on pharmaceutical container 40 to ensure the prescription was filled correctly and dispensed to the correct patient. Similarly, a patient may use the identifying images on pharmaceutical container 40 to ensure the prescriptions was filled correctly and dispensed to the correct patient. It will be understood that third parties may also use the identifying images on pharmaceutical container 40 to ensure the prescription was filled and dispensed accurately.

A pharmacist may also use an independent source to verify that the correct pharmaceutical was used to fill a prescription. For example, the pharmacist may access an independent reference, such as the physician's desk reference, to confirm that pharmaceutical-identifying image 34 corresponds to the prescribed pharmaceutical. As a further measure for ensuring accuracy, the pharmacist may access the physician's records to confirm that the prescription that the pharmacist is filling was indeed prescribed by the physician and that the pharmacist correctly filled the prescription. A pharmacist may also verify the patient-identifying image 36 printed on label 30 by using an independent source. For example, the pharmacist may request a driver's license or other form of photo identification. Additionally, the pharmacist may access an independently maintained database, which includes patient images.

Visual confirmation that the correct patient is taking the correct prescription pharmaceutical may be useful at home as well as at a health care provider's facility. For example, a patient at home reaching for a prescription from a medicine cabinet full of prescription containers may visually verify that the correct prescription container is retrieved by checking patient-identifying image 36 on label 30. Similarly, a nurse in a hospital or nursing home may visually verify that the correct prescription container is dispensed to a patient by checking patient-identifying image 36, or verify potential allergies or interactions by checking warning-identifying image 38.

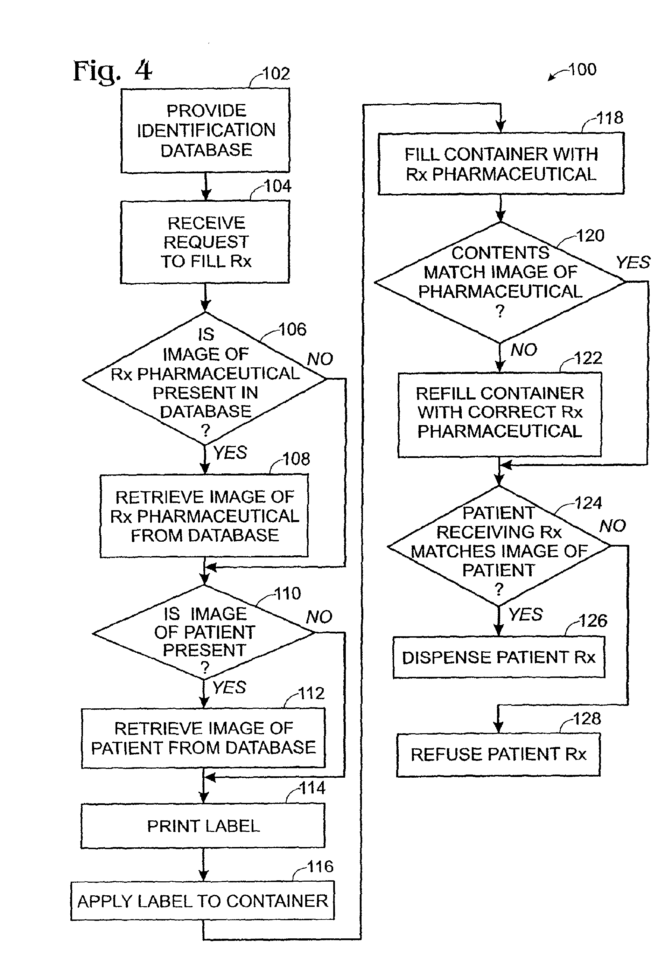

Fig. 4, is a flow chart which schematically illustrates, at 100, a method of using system 10 to verify that prescriptions are accurately filled and dispensed to the correct patient. Method 100 includes providing an identification database, as shown at 102. The identification database may include a patient database and/or a pharmaceutical database. The patient database may include patient-identifying images and other patient specific information such as name, address, age, sex, known drug allergies, etc. The pharmaceutical database may include pharmaceutical-identifying images and other pharmaceutical specific information such as black-box warnings, drug-to-drug interactions, possible side effects, etc.

Each database may be accessed proximately or remotely through a secure communications link. As noted above, the identification database may include both the patient database and the pharmaceutical database. It will be understood that the patient database and pharmaceutical database may be stored on the same server or terminal. However, each also may be stored on a separate terminal or server, or distributed across a plurality of terminals or servers. A physician may maintain a patient database on the physician's terminal or a separate server. Therefore, reference to the identification database refers to the patient database and/or the pharmaceutical database regardless of the actual location of the specific database.

A pharmacist receives a request to fill a prescription (Rx), as shown at 104. The request received by the pharmacist typically specifies a patient and a prescription pharmaceutical. Additionally, the request may include information about the patient receiving the prescription, the prescribed dosage of the pharmaceutical, the physician who wrote the prescription, and the number of refills. The prescription also may include a physician-specific code that permits a pharmacist to securely access a patient database stored on a specific physician's terminal or server.

Using a pharmacy terminal, the pharmacist accesses the identification database and checks for an identifying image of the specified pharmaceutical, at 106. Information about the specified prescription pharmaceutical may be retrieved and reviewed by the pharmacist. If the check indicates that there is a pharmaceutical-identifying image present in the identification database, that image may be retrieved, as shown at 108.

A pharmacist also may check the identification database for an image of the patient indicated in the prescription the pharmacist received, as shown at 110. It will be understood that the pharmacist may securely access a patient database stored on a physician's terminal or server using a physician-specific code provided on the prescription from the physician. If the check indicates an image of the patient is present, the image may be retrieved, as shown at 112. If a patient image is not present, a patient image may be captured and added to the identification database (not shown).

A custom prescription-specific pharmaceutical label thus may be printed including any retrieved identifying images, as shown at 114. The custom prescription pharmaceutical label may include the following information: the prescribing physician's name, the prescribing physician's business address, the patient's name, the patient's address, the pharmacy's name and address, the date the prescription was last filled, refill status, brief instructions for taking the pharmaceutical, the name of the pharmaceutical, the manufacturer of the pharmaceutical, and the prescribed dosage of the pharmaceutical. Any subset, or combination of the above mentioned information may be included on the prescription-specific label printed. Additionally, other information not included in the above list may be included on the label.

The label may include a peel-away release liner and adhesive layer for affixing the label to a pharmaceutical container. Alternatively, or additionally, tape of some other adhesive material may be employed to affix the label to the pharmaceutical container. The label also may be printed directly on the container, or on packaging of the pharmaceutical. The pharmacist thus may affix the printed label on the pharmaceutical container, as indicated at 116.

Once the custom prescription-specific pharmaceutical label has been affixed to the pharmaceutical container, the container is ready to be filled with the prescribed pharmaceutical. The pharmacist thus may place the prescribed pharmaceutical in the pharmaceutical container, at 118.

Once the container is filled, the contents of the container may be verified against the image of the pharmaceutical on the label to ensure that the prescription was accurately filled, as indicated at 120. If the appearance of the contents of the container does not match the image of the pharmaceutical on the label, then the prescription may be refilled in accordance with pharmacy procedures, at 122.

The patient receiving the prescription also may be verified by checking the appearance of the person picking up the prescription with the image of the patient on the label, at 124. If a person other than the patient is picking up the prescription, the pharmacist may ask for some form of identification, or other means, to confirm that the person picking up the prescription is acting on behalf of the correct patient. If the pharmacist is satisfied that the correct patient is going to receive the prescription pharmaceutical, the pharmacist may dispense the prescription to the patient, at 126. If the pharmacist is not satisfied that the correct patient is going to receive the prescription pharmaceutical the pharmacist may refuse to dispense the prescription pharmaceutical, at 128.

It should be understood that while the preceding method description focused on the implementation of the present invention in the context of filling prescriptions, a person of ordinary skill in the art would understand that this kind of verification system could be employed in any number situations requiring a custom label.

While the present invention has been particularly shown and described with reference to the foregoing preferred embodiments, those skilled in the art will understand that many variations may be made therein without departing from the spirit and scope of the invention as defined in the following claims. The description of the invention should be understood to include all novel and non-obvious combinations of elements described herein, and claims may be presented in this or a later application to any novel and non-obvious combination of these elements. The foregoing embodiments are illustrative, and no single feature or element is essential to all possible combinations that may be claimed in this, or a later application. Where the claims recite "a" or "a first" element or the equivalent thereof, such claims should be understood to include incorporation of one or more such elements, neither requiring nor excluding two or more such elements.

## Claims

1. A method of providing a pharmaceutical label, comprising: receiving a request (104) to fill a patient prescription for a pharmaceutical; accessing an identification database (106, 110) including identifying images; retrieving a prescription-specific identifying image (108, 112) from the identification database; and printing a pharmaceutical label (114) including the prescription-specific identifying image.

2. The method of claim 1, wherein the identifying images include images of patients, and wherein the prescription-specific identifying image (32) printed on the pharmaceutical label is an image of a patient (36) to receive the prescription.

3. The method of claim 1, wherein the identifying images include images of pharmaceuticals, and wherein the prescription-specific identifying image (32) printed on the pharmaceutical label is an image of pharmaceutical (38) which interacts with the prescribed pharmaceutical.

4. A system for managing prescription pharmaceutical transactions, the system comprising: memory (24) configured to store identifying images; a processor (23) configured to accommodate identification of a prescription pharmaceutical transaction, and to access the memory (24) to retrieve a prescription-specific identifying image (32); and a printer (16) configured to print a pharmaceutical label (30) including the prescription-specific identifying image (32).

5. The system of claim 4, wherein the memory (24) stores patient images, and wherein the prescription-specific identifying image is an image of a patient (36) to receive the prescription.

6. A pharmaceutical label produced by a label-making process comprising: receiving a request to fill a prescription (104) of a specific patient for a pharmaceutical; accessing an identification database (110) including images identifying patients; retrieving an identifying image (112) from the identification database which corresponds to the specific patient; and printing (114) the retrieved identifying image.

7. A pharmaceutical label produced by a label-making process comprising: receiving a request to fill a prescription (104) of a specific patient for a pharmaceutical; accessing an identification database (106, 110) including pharmaceutical-identifying images identifying pharmaceuticals and patient-identifying images identifying patients; retrieving a pharmaceutical-identifying image from the identification database which corresponds to the prescribed pharmaceutical (108) and retrieving a patient-identifying image (112) which corresponds to the specific patient; and printing the retrieved pharmaceutical-identifying image and patient-identifying image( 114).

8. A prescription pharmaceutical label comprising: a text portion (31,33,35,37,39) including textual pharmaceutical and patient information; and an image portion (32) including an identifying patient image (36) for confirming proper delivery of a prescribed pharmaceutical to a patient by visually comparing the identifying patient image (36) to a patient receiving the prescribed pharmaceutical.

9. A method for verifying that pharmaceutical prescriptions are filled accurately, comprising: receiving a request to fill a prescription (104) of a specified patient for a specified pharmaceutical; placing the specified pharmaceutical in a container (118) according to the received request to fill the prescription of the specified patient; accessing a prescription pharmaceutical database (106) including identifying images of prescription pharmaceuticals to retrieve an identifying image of the specified pharmaceutical from the prescription pharmaceutical database; printing the identifying image of the specified pharmaceutical on a label (114); applying the label to the container(116); verifying the contents of the container (120) by comparing the contents of the container to the identifying image of the specified pharmaceutical; accessing a patient database (110) including identifying images of patients to retrieve (112) an identifying image of the specified patient; printing the identifying image of the specified patient on the label (114); and verifying the identity of the patient (124) receiving the pharmaceutical by comparing an appearance of the patient receiving the pharmaceutical to the identifying image of the specified patient on the printed label.

10. A method for verifying that pharmaceutical prescriptions are filled accurately, comprising: providing a server (102) including a pharmaceutical database with identifying images of pharmaceuticals; receiving a request to fill a prescription (104) of a specified patient for a specified pharmaceutical; entering prescription information including the specified pharmaceutical and specified patient into a terminal linked to the server; accessing the server (106) to retrieve an identifying image of the specified pharmaceutical from the pharmaceutical database; printing a label (114) including the identifying image of the specific pharmaceutical; placing the specified pharmaceutical in a container (118) according to the request; applying the label to the container (116); verifying the contents of the container(120) by comparing the contents of the container to the identifying image of the specific pharmaceutical printed on the label; providing on the server (102) a patient database with identifying images of patients; accessing the patient database (110) of the server to retrieve an identifying image of the specified patient; verifying the identity of the patient receiving the pharmaceutical (124) by comparing an appearance of the patient receiving the pharmaceutical to the identifying image of the specific patient on the printed label.
